(19) Europäisches Patentamt
European Patent Office
Office européen des brevets

(11) **EP 2 567 199 B1**

# (12) EUROPEAN PATENT SPECIFICATION

(45) Date of publication and mention
of the grant of the patent:
**20.07.2016 Bulletin 2016/29**

(21) Application number: **11777354.9**

(22) Date of filing: **04.04.2011**

(51) Int Cl.:
*G01F 1/00* (2006.01)      *G01F 25/00* (2006.01)
*A61M 25/00* (2006.01)      *G01F 1/68* (2006.01)
*G01F 3/36* (2006.01)      *G01F 5/00* (2006.01)
*A61B 5/20* (2006.01)

(86) International application number:
**PCT/IL2011/000289**

(87) International publication number:
**WO 2011/138774 (10.11.2011 Gazette 2011/45)**

(54) **FLOW RATE METER**

FLUSSRATENREGLER

DÉBITMÈTRE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **03.05.2010 IL 20561410**

(43) Date of publication of application:
**13.03.2013 Bulletin 2013/11**

(73) Proprietor: **Renalsense Ltd.
Jerusalem 91450 (IL)**

(72) Inventors:
• **GRINSTEIN, Mor
71708 Modi'in (IL)**
• **MANTINBAND, Jack Yehoshua
90435 Efrata (IL)**

• **BENTOV, Shmuel
99850 D.N. HaElla (IL)**
• **ADLER, Michael
25147 Kfar Vradim (IL)**

(74) Representative: **Perani, Aurelio et al
Perani & Partners
Piazza San Babila, 5
20122 Milano (IT)**

(56) References cited:
**WO-A1-99/06800          WO-A1-2004/100788
WO-A2-2009/064984     US-A- 5 062 304
US-A- 5 170 656          US-A1- 2006 081 045
US-A1- 2009 314 101     US-A1- 2009 314 101**

## Description

### Field of the Invention

The present invention relates to the field of flow rate meters. Specifically, the invention relates to flow rate meters which enable the accurate determination of the volumetric flow rate of a liquid.

### Background of the Invention

Flow measurement is important in many fields. For example, many industrial processes require measurement of flow through various conduits in order to control the process appropriately. Other uses requiring measurement of a liquid or gas include delivery of a product to a consumer, such as gas, oil, and water. In the medical field, liquid measurement is sometimes applied to a patient's urine output.

Most flow measurement systems rely upon various assumptions regarding the properties of the liquid to be measured and will not work or must be adjusted to cope with deviations from the assumed properties. For example, one well-known technique applies thermal transfer principles applying King's Law to determine the flow rate. With this approach, the thermal properties of the liquid to be measured must be known in advance.

Thermal transfer flow meters typically measure flow continuously using a heating element and two temperature sensors (one upstream & one downstream from, or adjacent to, the heater). By measuring the temperature differential between the two thermometers, the flow is calculated. Alternatively, the temperature is kept constant at the heater and the energy required to do so is monitored, from which the flow can be calculated.

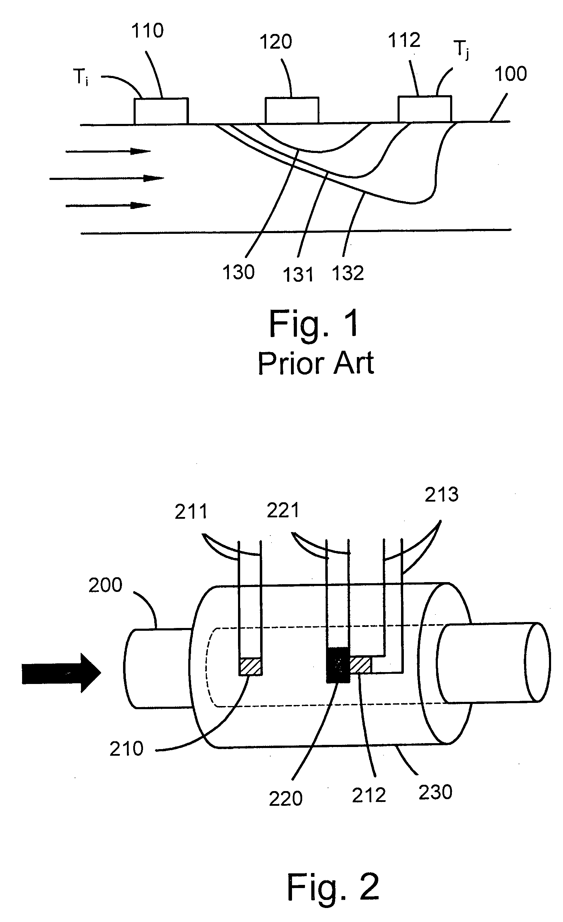

Fig.1 schematically shows the basic arrangement of a prior art thermal mass flow meter. A liquid flows through a tube 100 in a direction indicated by the arrows. At some location in wall of the tube is placed heating element 120 with temperature sensor 110, which measures temperature Ti, and temperature sensor 112, which measures temperature Tj, located respectively upstream and downstream of heater 120. Isothermal lines 130, 131, and 132 symbolically show the temperature distribution as a result of the power input to the heating element, where the $T_{130} > T_{131} > T_{132}$.

The calculation for determining the flow rate is according to the formula:

$$Q = C_p \cdot \rho \cdot V \cdot \Delta T \qquad \text{equation 1}$$

Noting that:

$$Q = I \bullet v \bullet t$$

Substituting and dividing both sides by t yields:

$$I \cdot v = C_p \cdot \rho \cdot \dot{V} \cdot \Delta T \qquad \text{equation 2}$$

And noting that

$$Q = I \bullet v \bullet t$$

**Solving for** $\dot{V}$ yields:

$$\dot{V} = (I \cdot v) \div (C_p \cdot \rho \cdot \Delta T) \qquad \text{equation 3}$$

wherein the symbols used herein are defined in the following table:

| Symbol | Meaning | Units |
|---|---|---|
| $V$ | Volume | [$l$] Liters |
| $\dot{V}$ | Volumetric Flow (volume/time) | $\left[\frac{l}{min}\right]$ Liters/minute |
| $Q$ | Energy, work | [$J$] Joules |
| $\rho$ | Density | $\left[\frac{g}{l}\right]$ grams/liter |
| $C_p$ | Specific Heat Capacity (under constant pressure) | $\left[\frac{J}{g \cdot \text{℃}}\right]$ Joules/(gram · ℃ ) |
| $T$ | Instantaneous Temperature | [°C] degrees Celsius |
| $T_i$ | Instantaneous Temperature of liquid before the heater (upstream) | [°C] degrees Celsius |
| $T_i$ | Instantaneous Temperature of liquid after or at the heater (downstream) | [°C] degrees Celsius |
| $I$ | Current | [$A$] Amperes |
| v | Electric potential | [v] Volts |
| $\Delta T$ | Temperature Difference $T_j$ - $T_i$ | [°C] degrees Celsius |
| $t$ | Time | [s] seconds |

A related type of thermal transfer flow meter, known, *inter alia,* as a constant temperature flow meter, uses a similar arrangement to that shown in Fig. 1 with the exception that temperature sensor 112 is adjacent to, or integral with heating element 120. In this configuration, the heating element 120 is heated to a set constant differential temperature $T_j$ (as measured by sensor 112) above the temperature Ti measured by sensor 110. As the flow varies, the amount of heat carried away by the flow varies. The temperature of heater 120 is kept constant by adjusting the current (assuming constant Voltage) applied thereto. The variation of the current required ($I$) to maintain a constant temperature differential $\Delta T$ provides a means to calculate the flow, as shown in equation 3.

As can be seen above, in order to accurately measure the flow rate using a thermal transfer flow meter, the density and the heat capacity of the measured liquid must be accurately known.

In some applications, there is no *a priori* knowledge of the liquid's properties, e.g. heat capacity and density. Some liquids can have varying properties - for example, urine is a liquid whose constituent components can vary from person to person, and, for a single person, can vary over time. As another example, milk can have varying fat content. In some applications, such as at a fuel terminal the same pipe may be used to transfer different types of fuel or gas or even sometimes either intentionally or unintentionally mixtures of gas and liquid products. In all of these situations, the readings of conventional thermal flow meters will be inaccurate and to improve the results the flow meters must be recalibrated on the basis of either assumptions that must be made about the properties of the liquid or empirical measurements.

In some cases, urine of bedside patients is measured manually, where urine flows along a catheter to a urine collection container and hospital personnel visually estimate the patient's urine output (ml/h) from the urine collection container. In practice, this arrangement is laborious and inaccurate, since hospital personnel must manually determine the amount of hourly urine and the dynamic nature of critical care settings makes it difficult to adhere to timely measurement. A simple, easy-to-use solution for measuring urine flow is needed to assist in accurate and timely measurement of urine output.

A brief understanding of related prior art can be gained from US 6,536,273, which discloses a thermal flow rate sensor that can be used with liquids of variable composition. The sensor comprises two elements: a conventional thermal flow sensor and a thermal-conductivity measuring cell. The thermal-conductivity measuring cell is used to determine the composition of the liquid and the results of measurements made from this cell are combined with other calibration measurements to correct the measurements made by the flow sensor for the properties of the liquid. WO 2004/100788 describes apparatus and a method for measuring urine flow taken out from a patient via a urinary tract catheter arrangement.

It is therefore an object of the invention to provide simple, cost-effective, and accurate flow rate meters, which enable measuring the flow rate of a liquid without knowing beforehand the (possibly dynamic) physicochemical characteristics of liquid being measured.

It is another object of the invention to provide medical systems comprising the flow rate meters of the invention which enables monitoring the flow rate of a biological liquid from a patient.

It is a further object of the invention to provide a method for determining the flow rate of a liquid without knowing the possibly dynamic physicochemical properties of the tested liquid beforehand.

Further purposes and advantages of this invention will appear as the description proceeds.

**Summary of the Invention**

In a first aspect the invention is an apparatus for measuring the volumetric rate of flow of a liquid having variable properties through a conduit comprising the following components:

  a) at least one heating or cooling element adapted to add or subtract a known quantity of heat to or from said liquid; and
  b) at least one temperature sensor adapted to measure the instantaneous temperature of said liquid; and
  c) one or more of the following:

   i. two valves located in said conduit and spaced apart by a distance, said valves creating a section of said conduit having a known volume in which said liquid can be isolated and held stationary;
   ii. a section of said conduit split into at least two sub-conduits that are hermetically attached at their upstream and downstream ends and a mechanism for managing the flow of liquid through said sub-conduits such that a known volume of said liquid can be isolated and held stationary in at least one of said sub-conduits;
   iii. a hollow inner chamber held in position inside said conduit by one or more supporting members and a solid cylindrical piston comprising a coaxial hole bored through its center; said hollow inner chamber closed on the upstream side with the exception of a coaxial entrance hole that is either opened or blocked by a valve that is activated by contact with said piston, which is adapted to be moveable in upstream and downstream directions within said conduit; wherein when said valve blocks said entrance hole of said inner chamber and said inner chamber contains a known volume of said liquid that is isolated and held stationary; and
   iv. a first segment of said conduit having a small cross section followed by a second segment of said conduit having a large cross section which is followed in turn by a third segment of conduit having a small cross section, wherein the ratio of the cross section of said second segment to that of said first and third segments is such that the velocity of the liquid flowing through said second segment is slowed significantly in relation to the velocities of the liquid flowing through said first and third segments;

  wherein, at least one of said heating or cooling elements and at least one of said temperature sensors is in thermal contact with said known volume of said liquid that is isolated and held stationary or is flowing with a significantly slowed velocity, thereby determining the value of an aggregate coefficient, which is equal to the density multiplied by the specific heat capacity of said liquid, directly from measurements of the instantaneous temperature, said known volume of liquid that is isolated and held stationary or is flowing with a significantly slowed velocity, and said known quantities of heat that are added to or removed from said known volume of said liquid that is isolated and held stationary or is flowing with a significantly slowed velocity; and

  wherein, at least one of said heating or cooling elements and at least one of said temperature sensors is in thermal contact with said liquid that is flowing through said conduit, thereby determining said volumetric rate of flow directly from measurements of the rate at which heat is added to or removed from said flowing liquid, measurements of the instantaneous temperature of said flowing liquid, and said aggregate coefficient, without the use of look-up tables, coefficients of a polynomial defining a correlation curve, or similar sources of information.

Embodiments of the apparatus of the invention comprise a control system that comprises at least one of the following components: a processor, input means, memory units, display devices, and output means. The components of the control system are configured to carry out at least one of the following:

  a) to activate at least one valve located in the conduit at a location at which it can be used to divert a known volume of liquid flowing in the conduit into the chamber and/or to hold the known volume of liquid stationary;
  b) to activate at least one heating or cooling element;
  c) to receive data from temperature sensors and other types of sensors or meters that are present in the apparatus;
  d) to use the received data to determine the volumetric flow rate;
  e) to store and display to a user information related to the operation of the apparatus and the properties of the liquid that are measured or determined by components of the apparatus;
f) to send instantaneous or historical values of measured temperatures and other information relative to the liquid and the apparatus to remote locations;
g) to send signals that can be used as input to other systems; and
h) to send alarms if there are predetermined changes in the flow rate or other measured properties of the liquid.

In embodiments of the apparatus comprising sub-conduits at least one of the sub-conduits may comprise sensors that are in contact with liquid flowing through or trapped in the sub-conduit and that are adapted to measure at least one of the following properties of the liquid: electrical conductivity, osmolarity, osmolality, pH, biomarkers, electrolytes, specific gravity, specific density, conductivity, presence and concentration of: creatinine, urea, uric acid, white blood cells, red blood cells, glucose, ketones, number/concentration of ions.

Embodiments of the apparatus of the invention may comprise at least one of:

a) a bubble trap located upstream of the measurement location;
b) a gas-permeable membrane located upstream of the measurement location;
c) a check valve located downstream of the measurement location; and
d) a check valve located upstream of the measurement location.

Embodiments of the apparatus of the invention may be adapted to be either connected to or an integral part of a catheter or a drainage tube leading from a patient. In embodiments of the invention urine flows through the catheter or drainage tube and the control system of the apparatus is adapted to monitor the urine temperature and to send an alarm if changes occur that exceed a predetermined rate.

In a second aspect the invention is a method for measuring the volumetric rate of flow of a liquid having variable properties through a conduit. The method comprises the following steps:

a) modifying a section of said conduit to form a chamber in which a known volume of said liquid can be isolated and either held stationary or in which the velocity of said liquid is reduced significantly ;
b) isolating and holding stationary or significantly slowing the velocity of a known volume of liquid in said chamber;
c) reading the temperature from a temperature sensor in thermal contact with the known volume of liquid that is isolated and held stationary or is flowing with a significantly slowed velocity to measure an initial temperature of said known volume of liquid;
d) activating a heating or cooling element in thermal contact with said known volume of liquid that is isolated and held stationary or is flowing with a significantly slowed velocity to add or remove a known quantity of heat to or from said known volume of liquid;
e) reading the temperature from a temperature sensor in thermal contact with said known volume of liquid that is isolated and held stationary or is flowing with a significantly slowed velocity to measure a final temperature of said liquid after said known quantity of heat has been added to or removed from said known volume of liquid;
f) determining the value of an aggregate coefficient, which is equal to the density multiplied by the specific heat capacity of said liquid, from said known volume of said liquid, said known quantity of heat, and the difference between said initial and said final temperatures;
g) activating a thermal flow meter comprising a heating or cooling element in thermal contact with said liquid flowing in said conduit, said heating or cooling element adapted to add or remove a known quantity of heat to or from said flowing liquid, and at least one temperature sensor in thermal contact with said liquid flowing in said conduit, said temperature sensor adapted to measure the instantaneous temperature of said flowing liquid;
h) determining said volumetric flow rate directly from measurements of the rate at which heat is added or removed from said flowing liquid, the value of said aggregate coefficient, and measurements of the instantaneous temperatures, without the use of look-up tables, coefficients of a polynomial defining a correlation curve, or similar sources of information.

In a third aspect of the invention, a method for measuring the volumetric rate of flow of a liquid having variable properties through a conduit is provided, the method comprising the following steps:

a) modifying a section of said conduit to form a chamber in which a known volume of said liquid can be isolated and either held stationary or in which the velocity of said liquid is reduced significantly;
b) isolating and holding stationary or significantly slowing the velocity of a known volume of liquid in said chamber;
c) reading the temperature from a temperature sensor in thermal contact with the known volume of liquid that is isolated and held stationary or is flowing with a significantly slowed velocity to measure an initial temperature of said known volume of liquid;

d) activating a heating or cooling element in thermal contact with said known volume of liquid that is isolated and held stationary or is flowing with a significantly slowed velocity and measuring the quantity of heat that is added or removed to or from said known volume of liquid;

e) reading the temperature from a temperature sensor in thermal contact with said known volume of liquid that is isolated and held stationary or is flowing with a significantly slowed velocity to determine when a predetermined final temperature is reached at which point said heating or cooling element is deactivated;

f) determining the value of an aggregate coefficient, which is equal to the density multiplied by the specific heat capacity of said liquid, from said known volume of said liquid, said measured quantity of heat, and the predetermined difference between said initial and said final temperatures;

g) activating a thermal flow meter comprising a heating or cooling element in thermal contact with said liquid flowing in said conduit, said heating or cooling element adapted to add or remove a known quantity of heat to or from said flowing liquid, and at least one temperature sensor in thermal contact with said liquid flowing in said conduit, said temperature sensor adapted to measure the instantaneous temperature of said flowing liquid;

h) determining said volumetric flow rate directly from measurements of the rate at which heat is added or removed from said flowing liquid, the value of said aggregate coefficient, and measurements of the instantaneous temperatures, without the use of look-up tables, coefficients of a polynomial defining a correlation curve, or similar sources of information.

In embodiments of the methods of the second or the third aspect, step b through step f are carried out simultaneously with step g and step h in different sections of the conduit.

In embodiments of the method of the second or the third aspect, the method is adapted to measure the volumetric flow of a liquid through a catheter or a drainage tube leading from a patient. In some of these embodiments the liquid is urine.

The present invention concerns also a method for measuring the volumetric rate of flow of a liquid having variable properties through a conduit, said method comprising the following steps:

a) modifying a section of said conduit to form a chamber in which a known volume of said liquid can be isolated and either held stationary or in which the velocity of said liquid is reduced significantly;

b) isolating and holding stationary or significantly slowing the velocity of a known volume of liquid in said chamber;

c) reading the temperature from a temperature sensor in thermal contact with the known volume of liquid that is isolated and held stationary or is flowing with a significantly slowed velocity to measure an initial temperature of said known volume of liquid;

d) activating a heating or cooling element in thermal contact with said known volume of liquid that is isolated and held stationary or is flowing with a significantly slowed velocity to add or remove heat to or from said known volume of liquid;

e) reading the temperature from a temperature sensor in thermal contact with said known volume of liquid that is isolated and held stationary or is flowing with a significantly reduced velocity to determine when a predetermined final temperature is reached at which point said heating or cooling element is deactivated;

f) resuming flow of fresh liquid through said location in the conduit that is adapted to isolate the liquid and to hold it stationary or to cause it to flow with a significantly slowed velocity and activating a timer simultaneously with the time at which said fresh liquid flow is initiated;

g) measuring the period of time that elapses until the temperature of the flowing liquid at said location shifts from said final temperature back to a threshold value of temperature; and

h) determining said volumetric flow rate from said known volume of said liquid and said measured period of time according to the equation volumetric flow rate equals known volume divided by measured period of time.

## Brief Description of the Drawings

The above and other characteristics and advantages of the invention will be more readily apparent through the following examples, and with reference to the appended drawings, wherein:

- Fig.1 schematically shows the basic arrangement of a prior art thermal transfer flow meter;
- Fig. 2 schematically shows a section of conduit comprising components used for measuring the flow of a liquid flowing through it using thermal transfer techniques;
- Fig. 3 schematically shows a section of a liquid conduit with a circular cross-section divided into two sub-conduits;
- Fig. 4 schematically shows a basic embodiment of a self-calibrating thermal mass flow meter according to the present invention;
- Figs. 5A and 5B schematically illustrate an embodiment in which at a certain point the conduit bifurcates into upper branch and lower branch, which rejoin at a location downstream;

- Figs. 5C, 5D, and 5E schematically illustrate embodiments of valves that are adapted to direct the flow from a main conduit to one of a plurality of sub-conduits while blocking the flow to the remaining sub-conduits;
- Figs. 6A and 6B schematically illustrate another embodiment of the flow measuring apparatus of the invention;
- Fig. 7 schematically illustrates a method of measuring the flow rate;
- Fig. 8 schematically illustrates an embodiment of the invention wherein the flow measurements and calibration can be carried out without stopping the flow of liquid through the conduit; and
- Fig. 9 schematically illustrates a system for measuring the flow of urine from a catheterized patient.

## Detailed Description of Preferred Embodiments

The present invention is flow rate meters which are able to accurately measure the volumetric flow rate of a liquid without requiring foreknowledge of the physicochemical characteristics of the liquid, e.g., for the purpose of calibration of the thermal mass flow rate. One application of the flow meters of the invention is to incorporate them in a system for measuring the flow rate of urine excreted by a catheterized patient. Other applications also exist and are included within the scope of this invention.

Consider now the heat capacity $C_p$ of the liquid flowing in a conduit. $C_p$ is defined as follows:

$$C_p = \frac{Q}{\rho V \Delta T}$$

which can be rearranged to yield:

$$\rho \bullet C_p = \frac{Q}{V \Delta T}$$

We now define an aggregate coefficient $y$, which represents the liquid properties.

$$y = \rho \bullet C_p$$

Substituting $y$ for $\rho \bullet C_p$, we have:

$$y = \frac{Q}{V \Delta T} \qquad\qquad \text{equation 4}$$

From equation 4 it can be appreciated that if:

- a known volume V of the liquid is isolated in a cell containing a heating element and temperature sensor; and
- the heat input $Q$ to the cell is known either though measurement or by dosing; and
- the change in temperature $\Delta T$ is known by measurement;

then sufficient information is available to determine $y$ without knowing the density or the specific heat capacity of the measured liquid individually.

We now substitute in equation 3 to obtain:

$$\dot{V} = (I \cdot v) \div (y \cdot \Delta T) \qquad\qquad \text{equation 5}$$

The values of $I$, $v$, $t$, and $\Delta T$ can be measured using a thermal flow meter as described with respect to Fig. 1. A known volume of the liquid can be isolated and measurements performed to measure the variable properties of the liquid flowing in a conduit, i.e. $y$. Then, using equation 5, a value of the flow rate V corrected for the variable properties of the liquid can be determined.

According to an embodiment, the apparatus is configured such that a portion of the conduit through which the liquid flows is adapted to allow part of the stream of liquid to be split off from the main stream and to enter a chamber that can be closed, thereby allowing determination of $y$. At the same time the liquid can continue flowing through the conduit and measurements can be made on the flowing liquid to obtain the rest of the data needed as input to equation 5. Alternatively, the chamber can be closed to measure the value of $y$ immediately after which the chamber is opened allowing measurements to be made on the flowing liquid to obtain the rest of the data needed using the same heating element and temperature sensors used to measure $y$.

It is important to realize that the method of the invention allows the actual value of the flow rate $\dot{V}$ to be determined directly using only measured parameters. This is as opposed to prior art methods of determining the mass flow rate that depend on look-up tables, coefficients of a polynomial defining a correlation curve, or similar sources of information to provide values based upon the density and specific heat capacity of the assumed composition of the liquid and typically derived by individual empirical calibration of the flow rate meter with the liquid of interest. The method of the invention also allows for the automatic selection of an appropriate set of correlation data to be applied based upon the measured value of $y$.

It is noted that heat capacity is an intrinsic property of the liquid under consideration. This property also varies with the pressure and temperature of the liquid, as well as its volume. When dealing with water, for example, the increase in volume is negligible. The coefficient of thermal expansion (CTE) for pure water at 20°C is 207 x 10-6 (0.000207; therefore if the temperature is raised by 10°C, the volume change is about 0.00207, which is about 0.2%. Thus, the method of the invention holds true for water and other liquids, e.g. urine, with negligible CTE. Similarly, the changes in $C_p$ for variations of temperature and pressure over appropriately chosen ranges are negligible.

Fig. 2 schematically shows a section of conduit 200 (e.g., tubing or pipe) comprising components used for measuring the thermal mass flow of a liquid flowing through it in the direction indicated by the arrow. A temperature sensor 210 is located upstream of a heating element 220. The temperature measured by sensor 210 can be read out to a control or display unit via the leads 211. Heating element 220 is located inside conduit 200 inserted directly into the flowing liquid or on a heat-conductive portion of wall of the conduit. Electric power is supplied to heating element 220 by applying a voltage via the leads 221. Adjacent to heating element 220 is a second temperature sensor 212 that measures the temperature of the liquid downstream of heating element 220. The temperature measured by sensor 212 is read out via the leads 213. Preferably the section of conduit that contains heating element 220 and temperature sensors 210,212 is thermally isolated from the surroundings using insulation 230.

Herein the various embodiments of the apparatus are generally described as comprising an electrical heating element, but the apparatus is not to be limited in this way. Alternate sources of heat can be used to raise the temperature of the liquid, for example ultrasound or radio frequency or other electromagnetic energy. In all embodiments described herein, instead of using a heating element to raise the temperature of the liquid for purposes of making the measurements equivalent results can be obtained by using a cooling element to lower the temperature of the liquid. Therefore, herein the use of terms such as "heating element" are to be understood as applying equally to "cooling elements" and *vice versa.* Cooling of the liquid at the location of the measurements can be accomplished by many different methods, e.g. use of a Peltier device, a fan, or a coil of tubing through which refrigerant or cold liquid is circulated.

Although the heating element and temperature sensor are described herein as separate elements for convenience of describing their respective functions, embodiments are possible wherein a single element, e.g. a self-heating thermistor or resistive thermal device (RTD), can be used to enable both the heating and the temperature measurement functions.

The apparatus shown in Fig. 2, can be used to determine flow as described above (prior art). However, in a similar way it can also be used to determine the newly defined coefficient $y$. In order to determine the value of $y$ a valve is provided to close the section of conduit 200 where the heat is applied and the temperature measurements are made so that a known volume of the liquid is trapped, isolated and stationary. When these conditions are provided, equation 4 can be used to calculate the coefficient y. This value is used to calibrate a thermal transfer meter, which may be located elsewhere on the conduit. In this way the thermal transfer meter can be periodically dynamically updated to accurately measure the flow of a liquid with time-varying properties. In addition to providing a means to compensate the flow measurements for any changes in the properties of the liquid, $y$ may also be of significance in various applications. For example, when measuring urine flow in a catheter, $y$ may have useful clinical significance as an indicator of patient health.

Consider a simple liquid conduit with liquid flowing through it from upstream to downstream. At some point the conduit can be split into two or more sub-conduits hermetically attached to the main conduit through which the liquid continues to flow. Further downstream the sub-conduits can be rejoined to again form a simple conduit. Also a mechanism can be introduced to manage the flow through the sub-conduits, such that at any given time, liquid does not have to be flowing through all of them. This is useful when it is desirable to isolate a portion of the liquid for any of various purposes, including but not limited to measuring properties of the liquid or altering properties of the isolated portion of the liquid. While some of the following examples discuss two parallel conduits, in practice any number of conduits may be used.

For example, the flow may be directed through a conduit used to analyze one set of liquid properties and then directed to a second conduit for a different purpose and then to a third for maintaining the flow while the first two conduits remain closed. In similar fashion, any number of samples may be isolated while maintaining flow through the system.

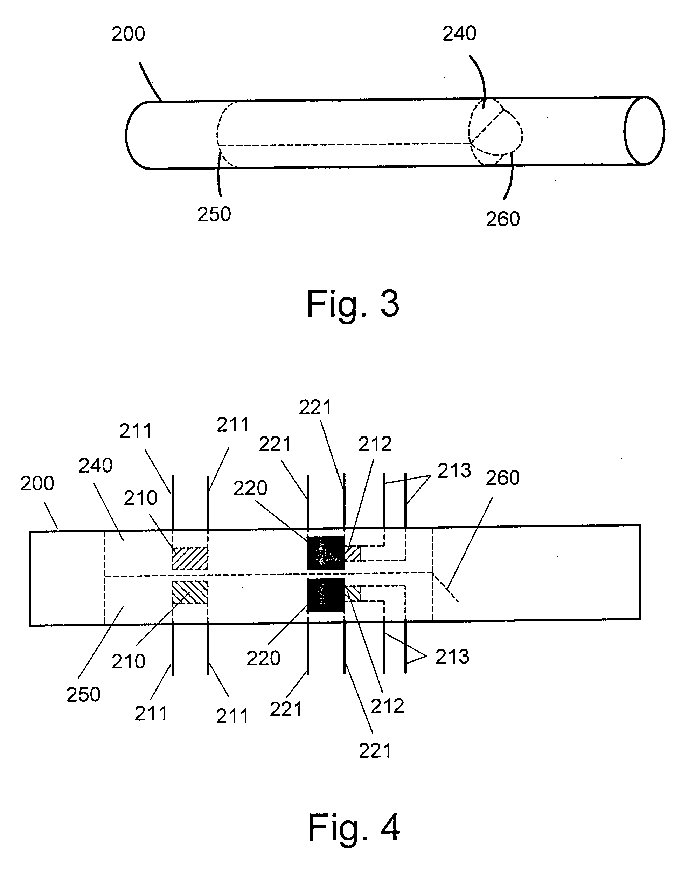

Fig. 3 schematically shows a section of a liquid conduit 200 with a circular cross-section. A section of the conduit is divided into an upper sub-conduit 240 and a lower sub-conduit 250. A movable flap 260 at one end of the divided section can be used to close one end of either the upper or lower sub-channels or neither one of them. When one sub-conduit is closed a known volume of liquid is trapped in it. At the same time that a quantity of liquid is trapped in one sub-conduit, the other sub-conduit is open, so the liquid can continue to flow. With this embodiment, the flap can be either at the upstream or downstream end of the sub-conduit - the flap, when closed, will trap the liquid in that sub-conduit by either preventing new liquid from entering or by preventing the trapped liquid from exiting. Of course, the two sub-channels may be in other configurations, e.g., side-by-side.

Fig. 4 schematically shows a basic embodiment of a self calibrating thermal mass flow meter according to the present disclosure. This embodiment employs the conduit shown in Fig. 3. The lower sub-conduit 250 will be used as the isolation chamber of known volume in which the measurements of $y$ will be made. Both upper sub-conduit 240 and lower sub-conduit 250 contain an arrangement of temperature sensors and heating element identical to that shown in Fig. 2. In embodiments the arrangement of temperature sensors and heating element that is located in upper sub-conduit 240 can be located at any place either upstream or downstream in the main conduit 200; however, especially if the flow rate is relatively high and/or the properties of the liquid are rapidly changing in time, the measurements in the flowing liquid and in the isolated liquid should be made physically and temporally close together to ensure accurate results.

In some applications it may be necessary to ensure that bubbles are eliminated from the liquid to ensure the conduit is full of liquid where the measurements are being made. In such an application one or more bubble traps may preferably be employed upstream of the measurement locations. Alternatively, means to allow gases to escape, such as a gas-permeable membrane may be located upstream of the measurement location.

At some conduit orientations and flow rates the conduit or sub-conduit may not be filled at the measurement location. Therefore, for some applications a check valve should be installed downstream of the measurement spot in order to create a sufficient back pressure to insure complete filling of the conduit at the location of the measurements. In some applications a check valve may be installed upstream of the measurement location, for example in order to prevent an automated system from trying to make measurements if the flow rate is below a certain value. For most applications a simple mechanical type check valve that opens when a preset pressure is exceeded is sufficient; however, in applications in which the orientation of the conduit and/or flow rate vary over a wide range of variables it is desirable to use a more sophisticated type check valve, e.g. one that is opened by a signal from a liquid level sensor in the conduit at the measurement location.

As mentioned previously, it is also possible to use the same set of heater and sensors for measuring $y$ when the liquid is trapped and then measuring flow when the liquid is not trapped, having taken into account any necessary recalibration due to the measured $y$. Also, in a situation where the flow can be temporarily stopped without harm, the above described system can be employed without a secondary sub-channel to maintain the flow. In situations where stopping the flow may cause harm, e.g., due to build-up of pressure, a secondary channel to maintain the flow obviates the problem.

All of the leads 211, 213, 221 are connected to a control system which comprises a processor adapted to activate the heating elements at predetermined times and voltage levels, to receive data from the temperature sensors, voltmeters, and ammeters and to use this data to determine the flow rate. The control system may also comprise means for opening and closing the flap to isolate part of the liquid, input means, e.g. a keypad, a keyboard, a touch screen, to allow a user to control parameters such as the length of time and/or quantity of heat energy that should be applied and the frequency with which the measurements are made. The control system can also comprise one or more memory units, display units, and output means to store and display to the user parameters of the system. The output means comprise communication devices that can be adapted to send the instantaneous or historical data to remote locations by using wired or wireless technologies. In addition the control system can be adapted to use the output means to send signals that provide input to other systems. For example, in an industrial setting, the signals can activate valves to cease the flow of a solution when a predetermined volume of a component has entered a mixing chamber.

In a hospital setting, the control system can be adapted to send an alarm to a nurse's station if the flow of urine from a catheterized patient to a collection bag falls below a predetermined rate, or to send an alarm if any irregularity occurs in the flow rate of a medicine being administered intravenously. When the apparatus is used for urine measurement, the temperature sensors report urine temperature. By itself that may not be useful, since the urine will have cooled off on the way to the sensor, but changes in the temperature can be very meaningful - e.g., going up by a degree or two can give immediate indication of a fever, without waiting for the periodic temperature check by the staff, which may be hours later. The control system of the invention can be adapted to monitor the urine temperature and to send an alarm to the nurse's station if changes occur that exceed a predetermined rate.

Many other arrangements for isolating a portion of the flowing liquid, whether by means of stopping the flow or diverting it, are also possible as will be obvious to one trained in the art. Such arrangements can take many forms. Some examples of different embodiments will now be described. The heating elements and temperature sensors have been omitted in the description of most of the embodiments herein below; however it is to be understood that they are present and are employed *mutatis mutandis* in similar locations and ways as has been described in relation to Fig. 4

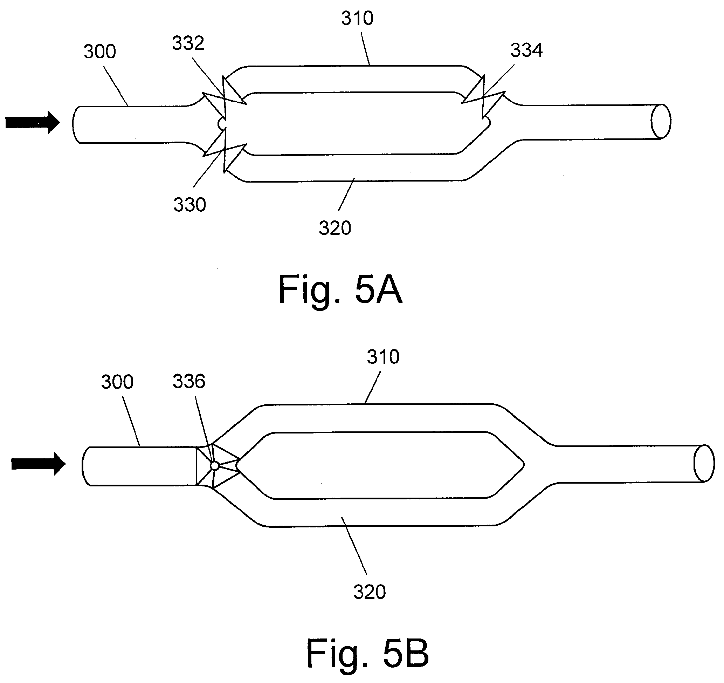

Figs. 5A and 5B schematically illustrate an embodiment in which at a certain point the conduit 300 bifurcates into upper branch 310 and lower branch 320, which rejoin at a location downstream. One, two, or three valves may be provided as shown in Fig. 5A. If only valve 330 is present, then when it is opened liquid flowing through conduit 300 continues flowing through both branches and when valve 330 is closed liquid continuously flows through branch 310 and the liquid in branch 320 is isolated, allowing the value of *y* to be measured. If both valves 330 and 332, are present then liquid can be isolated in either branch 310 or in branch 320. Valve 334 closed in conjunction with valve 332 guarantees that the liquid isolated in upper branch 310 is not in any way contacted by liquid flowing in the remainder of the system. This provides a location, i.e., in branch 310, in which to measure *y* in complete isolation from the flowing liquid. In embodiments of the present disclosure, when measurements are to be made, valve 334 can be closed first and valve 332 is closed a predetermined period later or only when sensors indicate that branch 310 is completely full with liquid. If there is a possibility that air can be in the pipe as well as liquid, an air vent to release trapped air can be located in branch 310. It is noted that when valve 334 is employed, measures may be required to account for thermal expansion, depending upon the application.

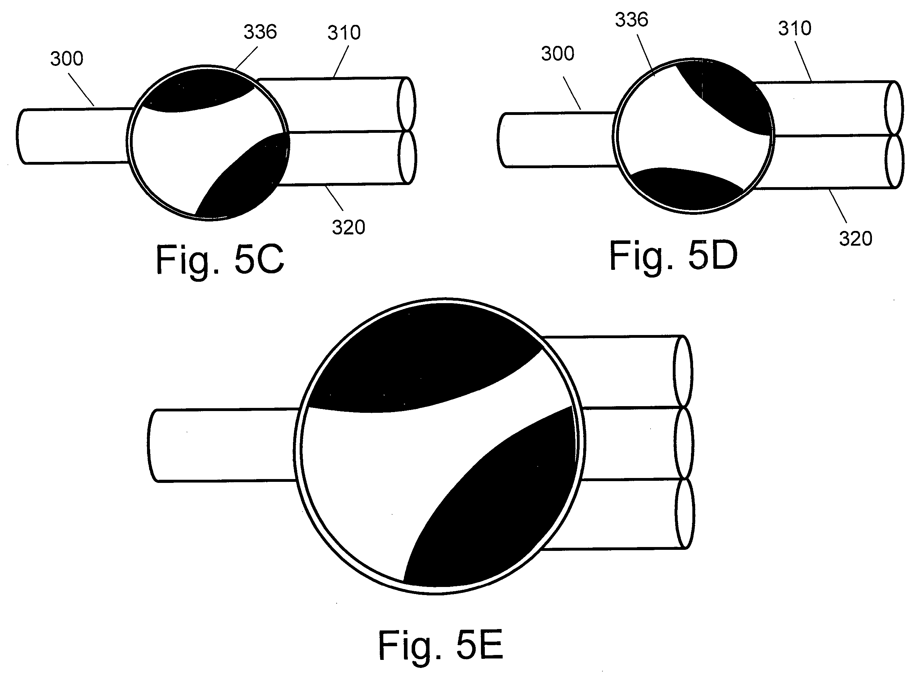

In the embodiment shown in Fig. 5B the configuration of the conduit is the same as in Fig. 5A however only one valve 336 is located in the main conduit 300 at the bifurcation. Valve 336, e.g., a rotating ball or cylindrical plug valve, is adapted to direct the flow into either one or the other of the branches 310,320 while closing the entrance to the other one, thereby isolating the liquid in it. Examples of a cross-section of valve 336 such as that described above are shown in the Figs. 5C and 5D. Other types of multiport valves may be employed as known in the art to accomplish the same purpose.

Fig. 5E schematically illustrates a valve that can be suited to directing the flow to only one out of three branches of a trifurcated channel. Many types of valves are commercially available or can be adapted by one trained in the art to meet the requirements for directing the flow from a main conduit to one of a plurality of sub-conduits while blocking the flow in the remaining sub-conduits. Multiple conduits for multiple samples are desirable when multiple types of measurements are to be made in parallel or series fashion. For example, depending on the liquid whose flow rate is being measured, sensors can be incorporated into the sub-conduits to measure some or all of the following properties of the liquid: electrical conductivity, osmolarity, osmolality, pH, biomarkers, electrolytes, specific gravity, specific density, thermal conductivity, presence and concentration of: creatinine, urea, uric acid, white blood cells, red blood cells, glucose, ketones, ions, e.g. $Na^+$, $K^+$, $Ca^{++}$, $Cl^-$.

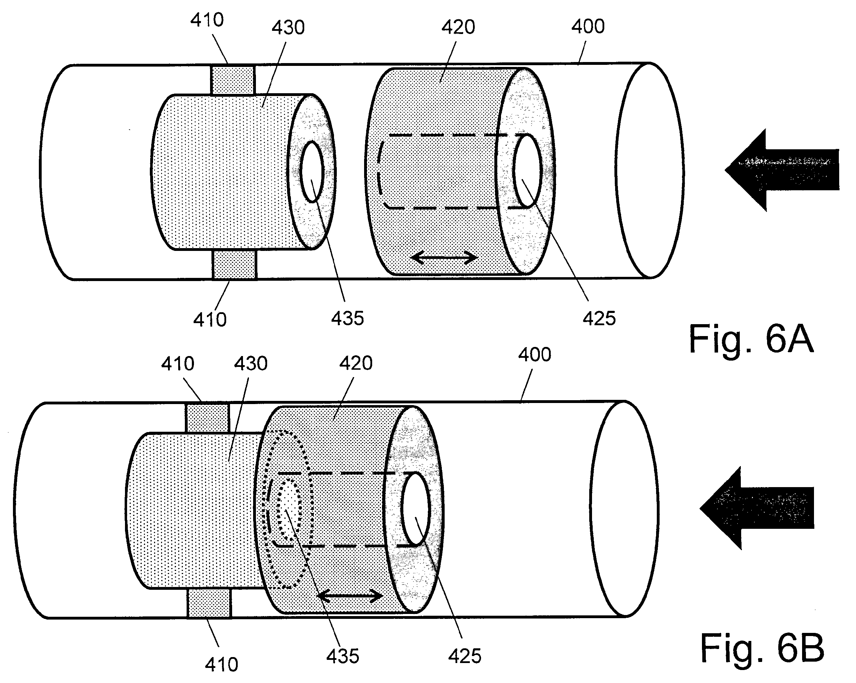

Figs. 6A and 6B schematically illustrate a flow measuring apparatus according to a first aspect of the invention. In the apparatus, liquid conduit 400 carries the liquid in the direction shown by the large arrow. Inside conduit 400 is located an inner chamber 430. Inner chamber 430 is cylindrically shaped to match the shape of conduit 400. It is hollow and is closed on the upstream side with the exception of a coaxial entrance hole that is either open or blocked by a normally closed valve 435. The inner chamber 430 is held in position by one or more supporting members 410 that are designed to hold inner chamber 430 in the correct position while offering minimal resistance to the flow of liquid around the outside of the inner chamber. Inner chamber 430 contains the heating element and temperature sensors used to make the measurements. Located inside of conduit 400 upstream of inner chamber 430 is a piston 420. Piston 420 is a solid cylinder with a coaxial hole 425 bored through its center. Piston 420 is constructed so as to allow the liquid to flow through the hole 425 but not around the piston between its outer surface and the inner surface of the wall of conduit 400. Piston 420 can be moved back and forth between the position shown in Fig. 6A and that shown in Fig. 6B. When piston 420 is in the position shown in Fig. 6B, valve 435 is open, allowing the liquid to flow through the inner chamber 430. In this configuration the flow measurements can be made. When piston 420 is in the position shown in Fig. 6A, the valve 435 is closed and the liquid flows around the outside of the inner chamber 430 while liquid that is inside the inner chamber 430 is trapped. In this configuration the measurements to determine parameter *y* that is used to calibrate the flow measurement can be made. An entry valve 435 with the properties described may be constructed with a ball and spring mechanism, a spring-loaded piston, or any of the other means known in the art. The piston 420 may be moved mechanically though the use of an electric coil, e.g., solenoid, nano-motors, or other means known in the art.

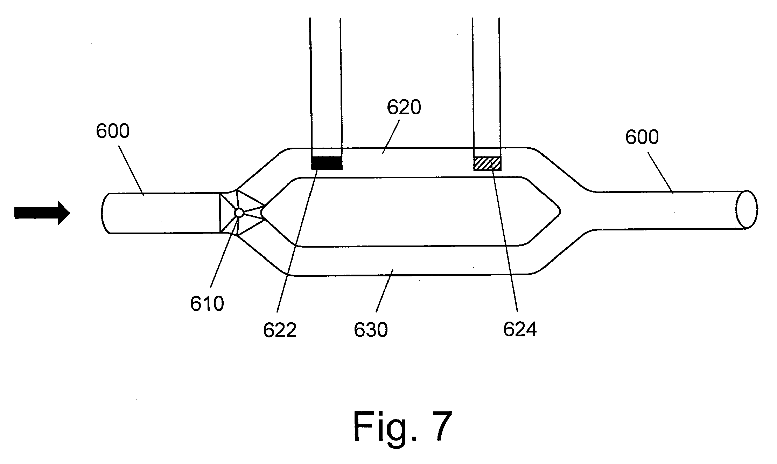

Fig. 7 schematically illustrates a method of directly measuring the flow rate without the need of determining and using a value of the coefficient *y* described herein above. Initially valve 610 closes branch 620 of conduit 600 trapping the liquid therein, while allowing liquid to continue to flow through branch 630. The trapped liquid in branch 620, which preferably is thermally insulated to prevent heat transfer to and from the surroundings, is at an initial temperature Ti as measured by temperature sensor 624. The trapped liquid is heated by the heater 622 until a given temperature, $T_j$, is read by temperature sensor 624. The valve **610** is then reversed so that the liquid flow resumes through branch 620 and branch 630 is closed. Simultaneously a timer is started. When a temperature shift from $T_j$ back to a threshold value of temperature that may be but is not necessarily Ti is measured by temperature sensor 624 the elapsed time is read from the timer. The time read, together with the known volume of the liquid displaced in branch 620 by liquid flowing into it from conduit 600 is used to calculate the sampled flow rate (volume/time). The threshold is a value that is between $T_i$ & $T_j$ that is "high enough" or "low enough" to indicate the transition of a quantity of liquid of interest. The use of a threshold is necessary because liquid doesn't get heated in a "rectangular" fashion and it may cool slightly as it flows - so the transition isn't a step function but is a gradient. For purposes of the flow calculation it is a matter of simple empirical testing depending on the application to determine the appropriate threshold to use. The threshold isn't merely a static temperature, but can also be a function of the rate of change, since different flows will affect it differently.

This method of measuring the flow rate makes it possible to keep track of the possibly changing flow rate without having to measure it continuously. Interpolation between the sample flow rates measured at different times is used to calculate the overall flow rate of the liquid through the conduit 600. The sampling process is repeated at a rate appropriate to the changing flow rate of the liquid and the accuracy requirements of the application. For example, a flow rate that changes frequently could be sampled more often, while a flow rate that changes less frequently could be sampled less often. If greater accuracy is required the flow rate can be sampled more frequently.

It is noted that according to this embodiment, the data gathered while the liquid is trapped in branch 620 can also be used to calculate y, which may be of clinical or other interest, but is not essential to determining the flow rate, which depends entirely on the displacement of the heated (or cooled) segment of liquid by liquid at its "natural" temperature, i.e. the liquid coming afterwards, which has not been heated or cooled.

It should be noted that this method described with Fig. 7 can be applied only when the temperature of the measured liquid does not change rapidly and by large amounts. For example, when making measurements of the flow rate of urine, if the temperature goes up or down due to a fever, the incremental change introduced by the heater 622 must be sufficiently larger than that caused by the fever so that the flow measurements will be accurate. Empirical testing can be used to verify that the proper conditions for using the method are met. Such empirical methods can be, for example, when the liquid is heated in chamber 620 observing the behavior of the cooling curve and how low the temperature gets. Also monitoring the starting temperature can be used. For example if the initial temperature $T_i$ is seen to be rising over time. In general, for urine measurements the temperature will not change so rapidly that the temperature of the urine that displaces the urine heated in branch 620 would be dramatically different from initial temperature Ti of the quantity of urine which was heated in the previous cycle.

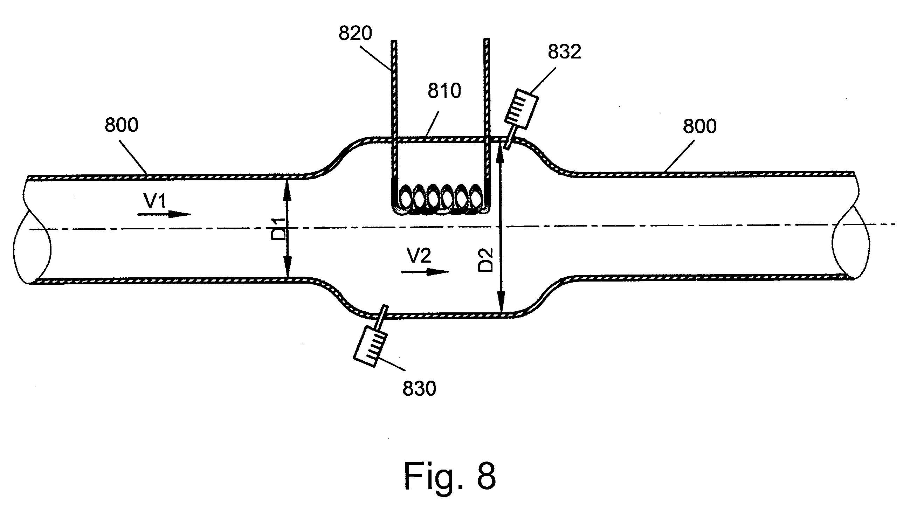

As an alternative to stopping the flow completely for purposes of measuring y, another embodiment of the invention entails slowing the velocity of the flow significantly in a region of the conduit, thus allowing the measurement to be made therein. Such an embodiment is illustrated in Fig. 8. As shown, the conduit has a small cross section segment 800 followed by a large cross section segment 810 which is followed in turn by a second small cross section segment. Also seen in the figure are heating element 820 and temperature sensors 830 and 832. For purposes of illustrating this embodiment, it is assumed the two small cross section segments are of the same diameter, D1 and the large cross section segment is of diameter D2. The conduit is configured to minimize turbulence at the transitions according to the principles known in the art and in accordance with the expected range of flow velocities to be handled. The flow velocity V2 through the large cross section segment 810 of the conduit will be slower than that of V1 through the small cross section segment 800, according to the inverse ratio of the squares of the diameters. Specifically, the velocity through the large cross section segment 810 will be in accordance with the following equation:

$$V2 = V1\frac{D1^2}{D2^2} \qquad \text{equation 6}$$

As can be seen, the conduit can be so configured as to achieve any desired slowing down of the liquid through the large cross section segment. For example, when the large cross section segment is of a diameter one order of magnitude greater than the small cross section segment, the velocity in the large cross section segment will be two orders of magnitude slower.

For purposes of illustration, consider the example where D1 is 3mm and D2 is 10mm and the flow rate is 1 cc/minute. The velocity V1 will be approximately 14.15 cm/minute. According to Equation 6, the velocity V2 in the large cross section segment will be approximately 1.273 cm/minute. If D2 is increased to 15mm, V2 is approximately 0.566 cm/min; for D2 = 20mm, V2 is approximately 0.318cc/min; and for D2 = 30 mm, $V_2$ is approximately 0.1415cm/minute.

Because the large cross section segment contains a relatively very slow-moving portion of the liquid, it may be treated as a chamber in which the liquid has been isolated, while taking into account the known parameters of the configuration and the consequences thereof with respect to the flow through the chamber during the measurement therein.

The measurement of y may be carried out in the chamber 810 so created according to the principles described above, with the following modifications: The amount of heat introduced into the chamber is a function of the time over which a pulse of energy is applied. Judicious timing of the pulse can be used to measure y. For example, applying a short, powerful pulse will yield a corresponding raise in temperature and y may be calculated in accordance with Equation 4, where the pulse's duration is short enough that the change in contents of the chamber is negligible. For example, in the last example cited above ($d_1$ = 3mm and $d_2$ = 30mm, flow rate of 1cc/minute), and using a chamber 810 of length 10mm, the volume of the chamber is approximately 7cm$^3$. Every second, the volume of liquid exchanged in the chamber is approximately 0.017cm$^3$, or less than 0.24% of the chamber volume. Thus during a pulse lasting 4 seconds or less, less than 1% of the chamber volume is exchanged. Therefore, the liquid within the chamber may be considered as standing still for an appropriately-timed measurement as described above.

As an additional consideration with regard to the previously described embodiments, while in certain applications it is preferable to have an apparatus where the flow continues unimpeded through an alternate conduit it is also possible to isolate the liquid by simply stopping the flow in a single conduit. In either case, operations may be performed on the isolated liquid in a portion of the closed conduit for the purposes of determining properties thereof and for deriving the flow rate of the liquid. The closed portion may be closed at one or both ends, depending on the needs of the application.

The above represent a few of the possible embodiments. There are many other embodiments of an apparatus to control the flow of liquid through a conduit such that one or more portions of the liquid may be isolated, as will be obvious to one trained in the art.

These embodiments can be provided as "built in" to dedicated flow lines or as separate units that can be incorporated into the flow line. For example to measure urine flow, the unit can have standard connections on both ends that allow it to be connected between the catheter and a collection bag or it may be incorporated in a catheter or in a drainage tube leading to a collection container.

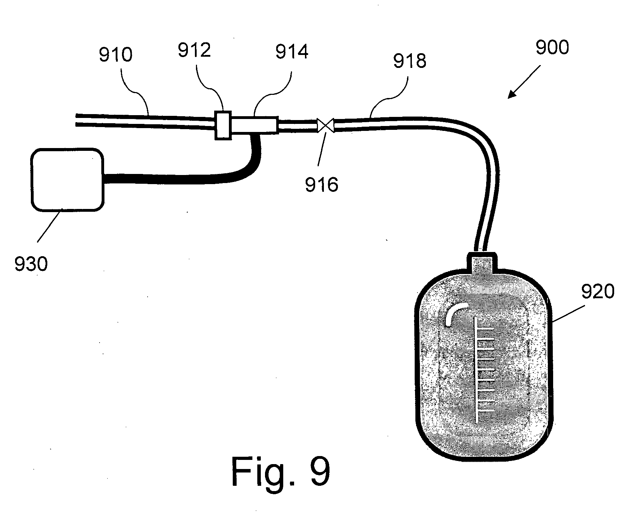

Fig. 9 schematically illustrates an embodiment of a system 900 for measuring the flow of urine from a catheterized patient (not shown in the figure). Shown in the figure are catheter 910, sensor unit 914, drain tube 918, collection bag 920, and control system 930. Optional components of system 900 are bubble trap 912 and check valve 916.

Sensor unit 914 represents the location where the measurements are made. Sensor unit 914 comprises one or more heating elements or cooling means and temperature sensors. It can be any one of the embodiments of the invention, examples of which are illustrated herein, e.g. as shown in Figs. 2 to 8.

Control system 930 comprises input means for inputting instructions and data, software and associated circuitry for activating the heater/cooler and temperature sensors in sensor unit 914, and, optionally, software and associated electrical circuitry and components for processing data obtained from the sensor unit 914, components for displaying the results on a visual display or as audible signals or communicating them to external systems.

**Claims**

**1.** An apparatus for measuring the volumetric rate of flow of a liquid having variable properties through a conduit (200,300,400,600,800), said apparatus comprising the following components:

a) at least one heating or cooling element (220,622,820) adapted to add or subtract a known quantity of heat to or from said liquid; and

b) at least one temperature sensor (210,212,624,830,832) adapted to measure the instantaneous temperature of said liquid; and

c) one or more of the following:

i. two valves located in said conduit (200) and spaced apart by a distance, said valves creating a section of said conduit (200) having a known volume in which said liquid can be isolated and held stationary;

ii. a section of said conduit (200,300,600) split into at least two sub-conduits (240,250,310,320,620,630) that are hermetically attached at their upstream and downstream ends and a mechanism (260,330,332,334,336,610) for managing the flow of liquid through said sub-conduits (240,250,310,320,620,630) such that a known volume of said liquid can be isolated and held stationary in at least one of said sub-conduits (240,250,310,320,620,630);

iii. a hollow inner chamber (430) held in position inside said conduit (400) by one or more supporting members (410) and a solid cylindrical piston (420) comprising a coaxial hole (425) bored through its center; said hollow inner chamber (430) closed on the upstream side with the exception of a coaxial entrance hole that is either opened or blocked by a valve (435) that is activated by contact with said piston (420), which is adapted to be moveable in upstream and downstream directions within said conduit (400); wherein when said valve (435) blocks said entrance hole of said inner chamber (430) and said inner chamber (430) contains a known volume of said liquid that is isolated and held stationary; and

iv. a first segment of said conduit (800) having a small cross section followed by a second segment of said conduit (810) having a large cross section which is followed in turn by a third segment of conduit (800) having a small cross section, wherein the ratio of the cross section of said second segment (810) to that of said first and third segments (800) is such that the velocity of the liquid flowing through said second segment (810) is slowed significantly in relation to the velocities of the liquid flowing through said first and third segments (800);

wherein, at least one of said heating or cooling elements (220,622,820) and at least one of said temperature sensors (210,212,624,830,832) is in thermal contact with said known volume of said liquid that is isolated and held stationary or is flowing with a significantly slowed velocity, thereby determining the value of an aggregate coefficient, which is equal to the density multiplied by the specific heat capacity of said liquid, directly from measurements of the instantaneous temperature, said known volume of liquid that is isolated and held stationary or is flowing with a significantly slowed velocity, and said known quantities of heat that are added to or removed from said known volume of said liquid that is isolated and held stationary or is flowing with a significantly slowed velocity; and

wherein, at least one of said heating or cooling elements and at least one of said temperature sensors is in thermal contact with said liquid that is flowing through said conduit, thereby determining said volumetric rate of flow directly from measurements of the rate at which heat is added to or removed from said flowing liquid, measurements of the instantaneous temperature of said flowing liquid, and said aggregate coefficient, without the use of look-up tables, coefficients of a polynomial defining a correlation curve, or similar sources of information.

2. The apparatus of claim 1, comprising a control system (930) that comprises at least one of the following components: a processor, input means, memory units, display devices, and output means, wherein said components of said control system(930) are configured to carry out at least one of the following:

a) to activate at least one valve (330,332,334,336,610) located in the conduit (300.600) at a location at which it can be used to divert a known volume of liquid flowing in said conduit (300,600) into a branch (310,320,620,630) of said conduit (300,600) and/or to hold said known volume of liquid stationary;
b) to activate at least one heating or cooling element (220,622,820);
c) to receive data from temperature sensors (210,220,624,830,832); heating or cooling elements (220,622,820); and other types of sensors or meters adapted to measure at least one of the following properties of said liquid: electrical conductivity, osmolarity, osmolality, pH, biomarkers, electrolytes, specific gravity, specific density, thermal conductivity, presence and concentration of: creatinine, urea, uric acid, white blood cells, red blood cells, glucose, ketones, number/concentration of ions that are present in said apparatus;
d) to use said received data to determine the volumetric flow rate;
e) to store and display to a user information related to the operation of said apparatus and the properties of said liquid that are measured or determined by components of said apparatus;
f) to send instantaneous or historical values of measured temperatures and other information relative to said liquid received from sensors adapted to measure at least one of the following properties of said liquid: electrical conductivity, osmolarity, osmolality, pH, biomarkers, electrolytes, specific gravity, specific density, thermal conductivity, presence and concentration of: creatinine, urea, uric acid, white blood cells, red blood cells, glucose, ketones, number/concentration of ions and said apparatus to remote locations;
g) to send signals that can be used as input to other systems; and
h) to send alarms if there are predetermined changes in the flow rate or other measured properties of said liquid.

3. The apparatus of claim 1.c.ii, wherein at least one of the sub-conduits (240,250,310,320,620,630) comprises sensors in contact with liquid flowing through or trapped in said sub-conduit (240,250,310,320,620,630), said sensors adapted to measure at least one of the following properties of said liquid: electrical conductivity, osmolarity, osmolality, pH, biomarkers, electrolytes, specific gravity, specific density, thermal conductivity, presence and concentration of: creatinine, urea, uric acid, white blood cells, red blood cells, glucose, ketones, number/concentration of ions.

4. The apparatus of claim 1 comprising at least one of:

a) a bubble trap (912) located upstream of the measurement location;
b) a gas-permeable membrane located upstream of said measurement location;
c) a check valve located downstream (916) of said measurement location; and
d) a check valve located upstream of said measurement location.

5. The apparatus of claim 1, wherein said apparatus is adapted to be either connected to or an integral part of a catheter (910) or a drainage tube (918) leading from a patient.

6. The apparatus of claim 5, wherein urine flows through the catheter (910) or drainage tube (920) and the control system (930) of said apparatus is adapted to monitor the urine temperature and to send an alarm if changes occur that exceed a predetermined rate.

7. A method for measuring the volumetric rate of flow of a liquid having variable properties through a conduit (200,300,400,600,800), said method comprising the following steps:

a) modifying a section of said conduit (200,300,400,600,800) to form a chamber in which a known volume of said liquid can be isolated and either held stationary or in which the velocity of said liquid is reduced significantly ;
b) isolating and holding stationary or significantly slowing the velocity of a known volume of liquid in said chamber;
c) reading the temperature from a temperature sensor (210,212,624,830,832) in thermal contact with the known volume of liquid that is isolated and held stationary or is flowing with a significantly slowed velocity to measure an initial temperature of said known volume of liquid;
d) activating a heating or cooling element (220,622,820) in thermal contact with said known volume of liquid that is isolated and held stationary or is flowing with a significantly slowed velocity to add or remove a known quantity of heat to or from said known volume of liquid;
e) reading the temperature from a temperature sensor (210,212,624,830,832) in thermal contact with said known volume of liquid that is isolated and held stationary or is flowing with a significantly slowed velocity to measure a final temperature of said liquid after said known quantity of heat has been added to or removed from said known volume of liquid;
f) determining the value of an aggregate coefficient, which is equal to the density multiplied by the specific heat capacity of said liquid, from said known volume of said liquid, said known quantity of heat, and the difference between said initial and said final temperatures;
g) activating a thermal flow meter comprising a heating or cooling element (220,622,820) in thermal contact with said liquid flowing in said conduit (200,300,400,600,800), said heating or cooling element (220,622,820) adapted to add or remove a known quantity of heat to or from said flowing liquid, and at least one temperature sensor (210,212,624,830,832) in thermal contact with said liquid flowing in said conduit (200,300,400,600,800), said temperature sensor (210,212,624,830,832) adapted to measure the instantaneous temperature of said flowing liquid;
h) determining said volumetric flow rate directly from measurements of the rate at which heat is added or removed from said flowing liquid, the value of said aggregate coefficient, and measurements of the instantaneous temperatures, without the use of look-up tables, coefficients of a polynomial defining a correlation curve, or similar sources of information.

8. A method for measuring the volumetric rate of flow of a liquid having variable properties through a conduit (200,300,400,600,800), said method comprising the following steps:

a) modifying a section of said conduit (200,300,400,600,800) to form a chamber in which a known volume of said liquid can be isolated and either held stationary or in which the velocity of said liquid is reduced significantly;
b) isolating and holding stationary or significantly slowing the velocity of a known volume of liquid in said chamber;
c) reading the temperature from a temperature sensor (210,212,624,830,832) in thermal contact with the known volume of liquid that is isolated and held stationary or is flowing with a significantly slowed velocity to measure an initial temperature of said known volume of liquid;
d) activating a heating or cooling element (220,622,820) in thermal contact with said known volume of liquid that is isolated and held stationary or is flowing with a significantly slowed velocity and measuring the quantity of heat that is added or removed to or from said known volume of liquid;
e) reading the temperature from a temperature sensor (210,212,624,830,832) in thermal contact with said known volume of liquid that is isolated and held stationary or is flowing with a significantly slowed velocity to determine when a predetermined final temperature is reached at which point said heating or cooling element is deactivated;
f) determining the value of an aggregate coefficient, which is equal to the density multiplied by the specific heat capacity of said liquid, from said known volume of said liquid, said measured quantity of heat, and the predetermined difference between said initial and said final temperatures;
g) activating a thermal flow meter comprising a heating or cooling element (220,622,820) in thermal contact with said liquid flowing in said conduit (200,300,400,600,800), said heating or cooling element adapted to add or remove a known quantity of heat to or from said flowing liquid, and at least one temperature sensor (210,212,624,830,832) in thermal contact with said liquid flowing in said conduit (200,300,400,600,800), said temperature sensor adapted to measure the instantaneous temperature of said flowing liquid;
h) determining said volumetric flow rate directly from measurements of the rate at which heat is added or removed from said flowing liquid, the value of said aggregate coefficient, and measurements of the instantaneous temperatures, without the use of look-up tables, coefficients of a polynomial defining a correlation curve, or similar sources of information.

9. The method of either one of claim 7 or claim 8, wherein step b through step f are carried out simultaneously with step g and step h in different sections of the conduit (200,300,400,600,800).

10. A method for measuring the volumetric rate of flow of a liquid having variable properties through a conduit (200,300,400,600,800), said method comprising the following steps:

a) modifying a section of said conduit (200,300,400,600,800) to form a chamber in which a known volume of said liquid can be isolated and either held stationary or in which the velocity of said liquid is reduced significantly;

b) isolating and holding stationary or significantly slowing the velocity of a known volume of liquid in said chamber;

c) reading the temperature from a temperature sensor (210,212,624,830,832) in thermal contact with the known volume of liquid that is isolated and held stationary or is flowing with a significantly slowed velocity to measure an initial temperature of said known volume of liquid;

d) activating a heating or cooling element (220,622,820) in thermal contact with said known volume of liquid that is isolated and held stationary or is flowing with a significantly slowed velocity to add or remove heat to or from said known volume of liquid;

e) reading the temperature from a temperature sensor (210,212,624,830,832) in thermal contact with said known volume of liquid that is isolated and held stationary or is flowing with a significantly reduced velocity to determine when a predetermined final temperature is reached at which point said heating or cooling element (220,622,820) is deactivated;

f) resuming flow of fresh liquid through said location in the conduit (200,300,400,600,800) that is adapted to isolate the liquid and to hold it stationary or to cause it to flow with a significantly slowed velocity and activating a timer simultaneously with the time at which said fresh liquid flow is initiated;

g) measuring the period of time that elapses until the temperature of the flowing liquid at said location shifts from said final temperature back to a threshold value of temperature; and

h) determining said volumetric flow rate from said known volume of said liquid and said measured period of time according to the equation volumetric flow rate equals known volume divided by measured period of time.

11. The method of either one of claim 7 or claim 8, wherein said method is adapted to measure the volumetric flow of a liquid through a catheter (910) or a drainage tube (918) leading from a patient.

12. The method of claim 11, wherein the liquid is urine.

## Patentansprüche

1. Vorrichtung zum Messen der volumetrischen Strömungsgeschwindigkeit einer Flüssigkeit mit veränderlichen Eigenschaften durch eine Leitung (200, 300, 400, 500, 600, 800), wobei die Vorrichtung folgende Bestandteile umfasst:

a) mindestens ein Heiz- oder Kühlelement (220,622,820), das dazu eingerichtet ist, eine bekannte Wärmemenge an die Flüssigkeit abzugeben oder ihr zu entziehen; und

b) mindestens einen Temperaturfühler (210,212,624,830,832), der dazu eingerichtet ist, die Momentantemperatur der Flüssigkeit zu messen; und

c) eines oder mehrere der folgenden:

i. zwei Ventile, die in der Leitung (200) angeordnet und voneinander beabstandet sind, wobei die Ventile einen Abschnitt der Leitung (200) mit einem bekannten Volumen bilden, in dem die Flüssigkeit isoliert und stationär gehalten werden kann;

ii. einen Abschnitt der Leitung (200,300,600), der in mindestens zwei Teilleitungen (240, 250, 310, 320, 620, 630) unterteilt ist, die hermetisch an ihren stromabwärtigen und stromaufwärtigen Enden angeschlossen sind, und einen Mechanismus (260, 330, 332, 334, 336, 610), um den Flüssigkeitsstrom derart durch die Teilleitungen (240, 250, 310, 320, 620, 630) zu leiten, dass ein bekanntes Flüssigkeitsvolumen in mindestens einer der Teilleitungen (240, 250, 310, 320, 620, 630) isoliert und stationär gehalten werden kann;

iii. eine hohle Innenkammer (430), die in der Leitung (400) durch ein oder mehrere Trägerelemente (410) und einen festen Zylinderkolben (420) in Position gehalten wird, umfassend ein durch ihren Mittelpunkt gebohrtes, koaxiales Loch (425); wobei die hohle Innenkammer (430) an der stromaufwärtigen Seite geschlossen ist mit Ausnahme des koaxialen Eingangsloches, das entweder geöffnet oder mit einem Ventil (435) gesperrt ist, das durch den Kontakt mit dem Kolben (420) betätigt wird, der dazu eingerichtet ist, stromaufwärts und stromabwärts innerhalb der Leitung (400) bewegbar zu sein; wobei das Ventil (435) das Eingangsloch der Innenkammer (430) sperrt und die Innenkammer (430) ein bekanntes Flüssigkeitsvolumen enthält, das isoliert und stationär gehalten wird; und

iv. ein erstes Segment der Leitung (800) mit einem kleinen Querschnitt, gefolgt von einem zweiten Segment der Leitung (810) mit einem großen Querschnitt, seinerseits gefolgt von einem dritten Segment der Leitung (800) mit einem kleinen Querschnitt, wobei das Verhältnis des Querschnitts der zweiten Segments (810) zu den des ersten und des dritten Segments (800) derart ist, dass die Geschwindigkeit der durch das zweite Segment (810) fließenden Flüssigkeit wesentlich verlangsamt ist im Verhältnis zu den Geschwindigkeiten der durch das erste und das dritte Segment (800) fließenden Flüssigkeit;

wobei mindestens eines der Heiz- oder Kühlelemente (220,622,820) und mindestens einer der Temperaturfühler (210,212,624,830,832) sich in thermischem Kontakt mit dem bekannten Flüssigkeitsvolumen befindet, das isoliert ist und stationär gehalten wird oder mit einer wesentlich verlangsamten Geschwindigkeit fließt und dabei den Wert eines Gesamtkoeffizienten, der gleich der Dichte multipliziert mit der spezifischen Wärmekapazität der Flüssigkeit ist, direkt durch Messungen der Momentantemperatur, des bekannten Flüssigkeitsvolumens, das isoliert ist und stationär gehalten wird oder mit einer wesentlich verlangsamten Geschwindigkeit fließt, und der bekannten Wärmemengen ermittelt, die an die Flüssigkeit, die isoliert ist und stationär gehalten wird oder mit einer wesentlich verlangsamten Geschwindigkeit fließt, abgegeben oder ihr entzogen werden; und

wobei mindestens eines der Heiz- oder Kühlelemente und mindestens einer der Temperaturfühler sich in thermischem Kontakt mit der Flüssigkeit befindet, die durch die Leitung fließt und dabei die volumetrische Strömungsgeschwindigkeit direkt durch Messungen der Geschwindigkeit, mit der Wärme an die fließende Flüssigkeit abgegeben oder ihr entzogen wird, Messungen der Momentantemperatur der fließenden Flüssigkeit und des Gesamtkoeffizienten ermittelt, ohne Verwendung von Nachschlagetabellen, Koeffizienten eines Polynoms zum Definieren einer Korrelationskurve oder ähnlicher Informationsquellen.

2. Vorrichtung nach Anspruch 1, umfassend ein Kontrollsystem (930), welches mindestens eines der folgenden Bestandteile umfasst: einen Rechner, Eingabemittel, Speichereinheiten, Anzeigeeinrichtungen und Ausgabemittel, wobei die Bestandteile des Kontrollsystems (930) entsprechend ausgebildet sind, um mindestens einen der folgenden auszuführen:

a) Betätigen mindestens eines Ventils (330,332,334,336,610), das an einer Stelle der Leitung (300,600) angeordnet ist, an der es verwendet werden kann, um ein bekanntes, in der Leitung (300,600) fließendes Flüssigkeitsvolumen in eine Abzweigung (310,320,620,630) der Leitung (300,600) umzuleiten und/oder das bekannte Flüssigkeitsvolumen stationär zu halten;

b) Betätigen mindestens eines Heiz- oder Kühlelements (220,622,820);

c) Empfangen von Daten von den Temperaturfühlern (210,220,624,830,832); dem Heiz- oder Kühlelement (220,622,820); und anderen Arten von Fühlern oder Messgeräten, die dazu eingerichtet sind, mindestens eine der folgenden Eigenschaften der Flüssigkeit zu messen: elektrische Leitfähigkeit, Osmolarität, Osmolalität, pH-Wert, Biomarker, Elektrolyte, spezifisches Gewicht, spezifische Dichte, Wärmeleitfähigkeit, Vorhandensein und Konzentration von: Kreatinin, Harnstoff, Harnsäure, weiße Blutkörperchen, rote Blutkörperchen, Glucose, Ketone, Anzahl/Konzentration der in der Vorrichtung vorhandenen Ionen;

d) Verwenden der empfangenen Daten zur Ermittlung der volumetrischen Strömungsgeschwindigkeit;

e) Speichern und einem Benutzer Anzeigen der Informationen im Zusammenhang mit dem Betrieb der Vorrichtung und der von den Bestandteilen der Vorrichtung gemessenen oder ermittelten Eigenschaften;

f) Fernübertragen von aktuellen oder historischen Werten der gemessenen Temperaturen oder anderer Informationen bezüglich der Flüssigkeit, die von den Fühlern empfangen werden, die zum Messen von mindestens einer der folgenden Eigenschaften der Flüssigkeit eingerichtet sind: elektrische Leitfähigkeit, Osmolarität, Osmolalität, pH-Wert, Biomarker, Elektrolyte, spezifisches Gewicht, spezifische Dichte, Wärmeleitfähigkeit, Vorhandensein und Konzentration von: Kreatinin, Harnstoff, Harnsäure, weiße Blutkörperchen, rote Blutkörperchen, Glucose, Ketone, Anzahl/Konzentration der in der Vorrichtung vorhandenen Ionen;

g) Senden von Signalen, die als Eingabe in andere Systeme verwendet werden können; und

h) Senden von Alarmen bei vorgegebenen Veränderungen der Strömungsgeschwindigkeit oder anderer gemessener Eigenschaften der Flüssigkeit.

**3.** Vorrichtung nach Anspruch 1.c.ii, wobei mindestens eine der Teilleitungen (240, 250, 310, 320, 620, 630) Fühler umfasst, die in Kontakt mit der Flüssigkeit sind, die durch die Teilleitung (240, 250, 310, 320, 620, 630) fließt oder darin eingeschlossen ist, wobei die Fühler zum Messen von mindestens einer der folgenden Eigenschaften der Flüssigkeit eingerichtet sind: elektrische Leitfähigkeit, Osmolarität, Osmolalität, pH-Wert, Biomarker, Elektrolyte, spezifisches Gewicht, spezifische Dichte, Wärmeleitfähigkeit, Vorhandensein und Konzentration von: Kreatinin, Harnstoff, Harnsäure, weiße Blutkörperchen, rote Blutkörperchen, Glucose, Ketone, Anzahl/Konzentration von Ionen.

**4.** Vorrichtung nach Anspruch 1, umfassend mindestens eines der folgenden:

a) eine Blasenfalle (912), die stromaufwärts der Messstelle angeordnet ist;
b) eine gasdurchlässige Membran, die stromaufwärts der Messstelle angeordnet ist;
c) ein Rückschlagventil (916), das stromabwärts der Messstelle angeordnet ist; und
d) ein Rückschlagventil, das stromaufwärts der Messstelle angeordnet ist.

**5.** Vorrichtung nach Anspruch 1, wobei die Vorrichtung dazu eingerichtet ist, entweder mit einem von einem Patienten wegführenden Katheter (910) oder Dränageschlauch (918) verbunden zu werden oder ein Bestandteil desselben zu sein.

**6.** Vorrichtung nach Anspruch 5, wobei Urin durch den Katheter (910) oder den Dränageschlauch (918) fließt und das Kontrollsystem (930) der Vorrichtung zum Überwachen der Urintemperatur und zum Senden eines Alarms eingerichtet ist, wenn Änderungen eintreten, die eine vorgegebene Geschwindigkeit überschreiten.

**7.** Verfahren zum Messen der volumetrischen Strömungsgeschwindigkeit einer Flüssigkeit mit veränderlichen Eigenschaften durch eine Leitung (200, 300, 400, 500, 600, 800), wobei das Verfahren folgende Schritte umfasst:

a) Ändern eines Abschnitts der Leitung (200, 300, 400, 500, 600, 800), um eine Kammer zu bilden, in der ein bekanntes Flüssigkeitsvolumen isoliert und entweder stationär gehalten werden kann oder in der die Geschwindigkeit der Flüssigkeit wesentlich reduziert ist;
b) Isolieren und Stationär-Halten oder wesentliches Verlangsamen der Geschwindigkeit eines bekannten Flüssigkeitsvolumens in der Kammer;
c) Lesen der Temperatur von einem Temperaturfühler (210,212,624,830,832) in thermischem Kontakt mit dem bekannten Flüssigkeitsvolumen, das isoliert und stationär gehalten wird oder mit einer wesentlich verlangsamten Geschwindigkeit fließt, um eine Anfangstemperatur des bekannten Flüssigkeitsvolumens zu messen;
d) Aktivieren eines Heiz- oder Kühlelements (220,622,820) in thermischem Kontakt mit dem bekannten Flüssigkeitsvolumen, das isoliert und stationär gehalten wird oder mit einer wesentlich verlangsamten Geschwindigkeit fließt, um eine bekannte Wärmemenge an ein bekanntes Flüssigkeitsvolumen abzugeben oder ihm zu entziehen;
e) Lesen der Temperatur von einem Temperaturfühler (210,212,624,830,832) in thermischem Kontakt mit dem bekannten Flüssigkeitsvolumen, das isoliert und stationär gehalten wird oder mit einer wesentlich verlangsamten Geschwindigkeit fließt, um eine Endtemperatur der Flüssigkeit zu messen, nachdem eine bekannte Wärmemenge an das bekannte Flüssigkeitsvolumen abgegeben oder ihm entzogen wurde;
f) Ermitteln des Wertes eines Gesamtkoeffizienten, der gleich der Dichte multipliziert mit der spezifischen Wärmekapazität der Flüssigkeit ist, aus dem bekannten Flüssigkeitsvolumen, der bekannten Wärmemenge und der Differenz zwischen der Anfangs- und der Endtemperatur;
g) Aktivieren eines thermischen Durchflussmessers, umfassend ein Heiz- oder Kühlelement (220,622,820) in thermischem Kontakt mit der in der Leitung (200, 300, 400, 500,600,800) fließenden Flüssigkeit, das Heiz- oder Kühlelement (220, 622, 820), das dazu eingerichtet ist, eine bekannte Wärmemenge an die fließende Flüssigkeit abzugeben oder ihr zu entziehen, und mindestens einen Temperaturfühler (210, 212, 624, 830, 832) in thermischem Kontakt mit der Flüssigkeit in der Leitung (200, 300, 400, 500, 600, 800), wobei der Temperaturfühler (210,212,624,830,832) dazu eingerichtet ist, die Momentantemperatur der fließenden Flüssigkeit zu messen;
h) Ermitteln der volumetrischen Strömungsgeschwindigkeit direkt durch Messungen der Geschwindigkeit, mit der Wärme an die fließende Flüssigkeit abgegeben oder ihr entzogen wird, des Wertes des Gesamtkoeffizienten und die Messungen der Momentantemperaturen ohne Verwendung von Nachschlagetabellen, Koeffizienten eines Polynoms zum Definieren einer Korrelationskurve oder ähnlicher Informationsquellen.

**8.** Verfahren zum Messen der volumetrischen Strömungsgeschwindigkeit einer Flüssigkeit mit veränderlichen Eigenschaften durch eine Leitung (200,300,400,600,800), wobei das Verfahren folgende Schritte umfasst:

a) Ändern eines Abschnitts der Leitung (200, 300, 400, 600, 800), um eine Kammer zu bilden, in der ein bekanntes Flüssigkeitsvolumen isoliert und entweder stationär gehalten werden kann oder in der die Geschwindigkeit der Flüssigkeit wesentlich reduziert wird;

b) Isolieren und Stationär-Halten oder wesentliches Verlangsamen der Geschwindigkeit eines bekannten Flüssigkeitsvolumens in der Kammer;

c) Lesen der Temperatur von einem Temperaturfühler (210,212,624,830,832) in thermischem Kontakt mit dem bekannten Flüssigkeitsvolumen, das isoliert und stationär gehalten wird oder mit einer wesentlich verlangsamten Geschwindigkeit fließt, um eine Anfangstemperatur des bekannten Flüssigkeitsvolumens zu messen;

d) Aktivieren eines Heiz- oder Kühlelements (220,622,820) in thermischem Kontakt mit dem bekannten Flüssigkeitsvolumen, das isoliert und stationär gehalten wird oder mit einer wesentlich verlangsamten Geschwindigkeit fließt, und Messen der Wärmemenge, die an das bekannte Flüssigkeitsvolumen abgegeben oder ihm entzogen wird;

c) Lesen der Temperatur von einem Temperaturfühler (210,212,624,830,832) in thermischem Kontakt mit dem bekannten Flüssigkeitsvolumen, das isoliert und stationär gehalten wird oder mit einer wesentlich verlangsamten Geschwindigkeit fließt, um festzustellen, wenn eine vorgegebene Endtemperatur erreicht wird, bei der das Heiz- oder Kühlelement deaktiviert wird;

f) Ermitteln des Wertes eines Gesamtkoeffizienten, der gleich der Dichte multipliziert mit der spezifischen Wärmekapazität der Flüssigkeit ist, aus dem bekannten Flüssigkeitsvolumen, der gemessenen Wärmemenge und der vorgegebenen Differenz zwischen der Anfangs- und der Endtemperatur;

g) Aktivieren eines thermischen Durchflussmessgeräts, umfassend ein Heiz- oder Kühlelement (220,622,820) in thermischem Kontakt mit in der Leitung (200,300,400,800) fließenden Flüssigkeit, das Heiz- oder Kühlelement (220,622,820), das dazu eingerichtet ist, eine bekannte Wärmemenge an die fließende Flüssigkeit abzugeben oder ihr zu entziehen, und mindestens einen Temperaturfühler (210,212,624,830,832) in thermischem Kontakt mit der Flüssigkeit in der Leitung (200,300,400,600,800), wobei der Temperaturfühler dazu eingerichtet ist, die Momentantemperatur der fließenden Flüssigkeit zu messen;

h) Ermitteln der volumetrischen Strömungsgeschwindigkeit direkt durch Messungen der Geschwindigkeit, mit der Wärme an die fließende Flüssigkeit abgegeben oder ihr entzogen wird, des Wertes des Gesamtkoeffizienten und Messungen der Momentantemperaturen ohne Verwendung von Nachschlagetabellen, Koeffizienten eines Polynoms zum Definieren einer Korrelationskurve oder ähnlicher Informationsquellen.

9. Verfahren nach Anspruch 7 oder 8, wobei die Schritte b bis f gleichzeitig mit den Schritten g und h in verschiedenen Abschnitten der Leitung (200,300,400,600,800) ausgeführt werden.

10. Verfahren zum Messen der volumetrischen Strömungsgeschwindigkeit einer Flüssigkeit mit veränderlichen Eigenschaften durch eine Leitung (200,300,400,600,800), wobei das Verfahren folgende Schritte umfasst:

a) Ändern eines Abschnitts der Leitung (200,300,400,600,800), um eine Kammer zu bilden, in der ein bekanntes Flüssigkeitsvolumen entweder isoliert und stationär gehalten werden kann oder in der die Geschwindigkeit der Flüssigkeit wesentlich reduziert ist;

b) Isolieren und Stationär-Halten oder wesentliches Verlangsamen der Geschwindigkeit eines bekannten Flüssigkeitsvolumens in der Kammer;

c) Lesen der Temperatur von einem Temperaturfühler (210,212,624,830,832) in thermischem Kontakt mit dem bekannten Flüssigkeitsvolumen, das isoliert und stationär gehalten wird oder mit einer wesentlich verlangsamten Geschwindigkeit fließt, um eine Anfangstemperatur des bekannten Flüssigkeitsvolumens zu messen;

d) Aktivieren eines Heiz- oder Kühlelements (220,622,820) in thermischem Kontakt mit dem bekannten Flüssigkeitsvolumen, das isoliert und stationär gehalten wird oder mit einer wesentlich verlangsamten Geschwindigkeit fließt, um eine bekannte Wärmemenge an das bekannte Flüssigkeitsvolumen abzugeben oder ihm zu entziehen;

e) Lesen der Temperatur von einem Temperaturfühler (210,212,624,830,832) in thermischem Kontakt mit dem bekannten Flüssigkeitsvolumen, das isoliert und stationär gehalten wird oder mit einer wesentlich verlangsamten Geschwindigkeit fließt, um festzustellen, wenn eine vorgegebene Endtemperatur erreicht wird, bei der das Heiz- oder Kühlelement (220,622,820) deaktiviert wird;

f) Wiederaufnehmen frischen Flüssigkeitsstroms durch die Stelle in der Leitung (200,300,400,600,800), die dazu eingerichtet ist, die Flüssigkeit zu isolieren und stationär zu halten oder zu veranlassen, mit wesentlich verlangsamter Geschwindigkeit zu fließen, und Aktivieren eines Zeitgebers simultan mit der Zeit, zu der der frische Flüssigkeitsstrom aufgenommen wird;

g) Messen der Zeitdauer, die verstreicht, bis die Temperatur der fließenden Flüssigkeit an der Stelle von der Endtemperatur wieder auf einen Temperaturschwellenwert zurückkehrt; und

h) Ermitteln der volumetrischen Strömungsgeschwindigkeit aus dem bekannten Flüssigkeitsvolumen und der gemessenen Zeitdauer gemäß der Gleichung volumetrische Strömungsgeschwindigkeit gleich bekanntes Volumen geteilt durch die gemessene Zeitdauer.

11. Verfahren nach Anspruch 7 oder 8, wobei das Verfahren dazu eingerichtet ist, die volumetrische Strömungsgeschwindigkeit durch einen Katheter (910) oder Dränageschlauch (918) zu messen, der von einem Patienten wegführt.

12. Verfahren nach Anspruch 11, wobei die Flüssigkeit Urin ist.

**Revendications**

1. Appareil pour mesurer le débit volumétrique d'un liquide ayant des propriétés variables à travers un conduit (200, 300, 400, 600, 800), ledit appareil comprenant les composants suivants :

   a) au moins un élément chauffant ou réfrigérant (220, 622, 820) adapté pour ajouter ou retirer une quantité de chaleur connue audit ou dudit liquide ; et
   b) au moins un capteur de température (210, 212, 624, 830, 832) adapté pour mesurer la température instantanée dudit liquide ; et
   c) un ou plusieurs des éléments suivants :

      i. deux vannes situées dans ledit conduit (200) et espacées d'une distance, lesdites vannes créant une section dudit conduit (200) ayant un volume connu dans lequel ledit liquide peut être isolé et maintenu immobile ;
      ii. une section dudit conduit (200, 300, 600) divisée en au moins deux sous-conduits (240, 250, 310, 320, 620, 630) qui sont attachés hermétiquement à leurs extrémités amont et aval et un mécanisme (260, 330, 332, 334, 336, 610) pour gérer l'écoulement de liquide à travers lesdits sous-conduits (240, 250, 310, 320, 620, 630) de manière qu'un volume connu dudit liquide peut être isolé et maintenu immobile dans au moins un desdits sous-conduits (240, 250, 310, 320, 620, 630) ;
      iii. une chambre intérieure creuse (430) maintenue en position à l'intérieur dudit conduit (400) par un ou plusieurs éléments de support (410) et un piston cylindrique plein (420) comprenant un trou coaxial (425) percé à travers son centre ; ladite chambre intérieure creuse (430) fermée du côté amont à l'exception d'un trou d'entrée coaxial qui est soit ouvert soit bloqué par une vanne (435) qui est activée par contact avec ledit piston (420), qui est adapté pour être mobile dans des directions amont et aval à l'intérieur dudit conduit (400) ; dans lequel quand ladite vanne (435) bloque ledit trou d'entrée de ladite chambre intérieure (430) et ladite chambre intérieure (430) contient un volume connu dudit liquide qui est isolé et maintenu immobile ; et
      iv. un premier segment dudit conduit (800) ayant une petite section transversale suivi par un deuxième segment dudit conduit (810) ayant une grande section transversale qui est suivi à son tour par un troisième segment de conduit (800) ayant une petite section transversale, dans lequel le rapport entre la section transversale dudit deuxième segment (810) et celle desdits premier et troisième segments (800) est tel que la vitesse du liquide s'écoulant à travers ledit deuxième segment (810) est considérablement ralentie par rapport aux vitesses du liquide s'écoulant à travers lesdits premier et troisième segments (800) ;

   dans lequel, au moins un desdits éléments chauffants ou réfrigérants (220, 622, 820) et au moins un desdits capteurs de température (210, 212, 624, 830, 832) est en contact thermique avec ledit volume connu dudit liquide qui est isolé et maintenu immobile ou s'écoule avec une vitesse considérablement ralentie, en déterminant ainsi la valeur d'un coefficient global, qui est égal à la densité multipliée par la capacité calorifique spécifique dudit liquide, directement à partir de mesures de la température instantanée, dudit volume connu de liquide qui est isolé et maintenu immobile ou s'écoule avec une vitesse considérablement ralentie, et desdites quantités de chaleur connues qui sont ajoutées ou retirées dudit volume connu dudit liquide qui est isolé et maintenu immobile ou s'écoule avec une vitesse considérablement ralentie ; et dans lequel, au moins un desdits éléments chauffants ou réfrigérants et au moins un desdits capteurs de température est en contact thermique avec ledit liquide qui s'écoule à travers ledit conduit, en déterminant ainsi ledit débit volumétrique directement à partir des mesures de la vitesse à laquelle la chaleur est ajoutée audit ou retirée dudit liquide en circulation, des mesures de la température instantanée dudit fluide en circulation et dudit coefficient global, sans l'utilisation de tables de recherche, de coefficients d'un polynôme définissant une courbe de corrélation ou de sources d'informations similaires.

**2.** Appareil selon la revendication 1, comprenant un système de commande (930) qui comprend au moins un des composants suivants : un processeur, des moyens d'entrée, des unités de mesure, des dispositifs d'affichage et des moyens de sortie, dans lequel lesdits composants dudit système de commande (930) sont configurés pour exécuter au moins une des opérations suivantes :

a) activer au moins une vanne (330, 332, 334, 336, 610) située dans le conduit (300, 600) dans un emplacement dans lequel elle peut être utilisée pour dévier un volume connu de liquide s'écoulant dans ledit conduit (300, 600) dans une branche (310, 320, 620, 630) dudit conduit (300, 600) et/ou pour maintenir ledit volume connu de liquide immobile ;

b) activer au moins un élément chauffant ou réfrigérant (220, 622, 820) ;

c) recevoir des données à partir de capteurs de température (210, 220, 624, 830, 832) ; d'éléments chauffants ou réfrigérants (220, 622, 820) ; et d'autres types de capteurs ou dispositifs de mesure adaptés pour mesurer au moins une des propriétés suivantes dudit liquide : conductivité électrique, osmolarité, osmolalité, pH, marqueurs biologiques, électrolytes, gravité spécifique, densité spécifique, conductivité thermique, présence et concentration de : créatinine, urée, acide urique, globules blancs, globules rouges, glucose, cétones, nombre/concentration d'ions qui sont présents dans ledit appareil ;

d) utiliser lesdites données reçues pour déterminer le débit volumétrique ;

e) stocker et afficher pour un utilisateur des informations relatives au fonctionnement dudit appareil et aux propriétés dudit liquide qui sont mesurées ou déterminées par des composants dudit appareil ;

f) transmettre des valeurs instantanées ou historiques de températures mesurées et d'autres informations relatives audit liquide reçues à partir de capteurs adaptés pour mesurer au moins une des propriétés suivantes dudit liquide : conductivité électrique, osmolarité, osmolalité, pH, marqueurs biologiques, électrolytes, gravité spécifique, densité spécifique, conductivité thermique, présence et concentration de : créatinine, urée, acide urique, globules blancs, globules rouges, glucose, cétones, nombre/concentration d'ions et ledit appareil vers des emplacements à distance ;

g) transmettre des signaux qui peuvent être utilisés comme des entrées pour d'autres systèmes ; et

h) envoyer des alarmes s'il existe des changements prédéterminés dans le débit ou d'autres propriétés mesurées dudit liquide.

**3.** Appareil selon la revendication 1, point c. ii, dans lequel au moins un des sous-conduits (240, 250, 310, 320, 620, 630) comprend des capteurs en contact avec le liquide s'écoulant à travers ou emprisonné dans ledit sous-conduit (240, 250, 310, 320, 620, 630), lesdits capteurs adaptés pour mesurer au moins une des propriétés suivantes dudit liquide : conductivité électrique, osmolarité, osmolalité, pH, marqueurs biologiques, électrolytes, gravité spécifique, densité spécifique, conductivité thermique, présence et concentration de : créatinine, urée, acide urique, globules blancs, globules rouges, glucose, cétones, nombre/concentration d'ions.

**4.** Appareil selon la revendication 1 comprenant au moins un de :

a) un piège à bulles (912) situé en amont de l'emplacement de mesure ;

b) une membrane perméable au gaz située en amont dudit emplacement de mesure ;

c) un clapet de retenue situé en aval (916) dudit emplacement de mesure ; et

d) un clapet de retenue situé en amont dudit emplacement de mesure.

**5.** Appareil selon la revendication 1, dans lequel ledit appareil est adapté pour être soit connecté à ou partie intégrante d'un cathéter (910) ou un tube de drainage (918) arrivant d'un patient.

**6.** Appareil selon la revendication 5, dans lequel urine s'écoule à travers le cathéter (910) ou le tube de drainage (920) et le système de commande (930) dudit appareil est adapté pour surveiller la température de l'urine et pour envoyer une alarme si des changements se produisent qui dépassent un taux prédéterminé.

**7.** Procédé pour mesurer le débit volumétrique d'un liquide ayant des propriétés variables à travers un conduit (200, 300, 400, 600, 800), ledit procédé comprenant les étapes suivantes :

a) la modification d'une section dudit conduit (200, 300, 400, 600, 800) pour former une chambre dans laquelle un volume connu dudit liquide peut être isolé et maintenu immobile ou dans laquelle la vitesse dudit liquide est considérablement réduite ;

b) l'isolement et le maintien immobile ou le ralentissement considérable de la vitesse d'un volume connu de liquide dans ladite chambre ;

c) la lecture de la température à partir d'un capteur de température (210, 212, 624, 830, 832) en contact thermique avec le volume connu de liquide qui est isolé et maintenu immobile ou s'écoule avec une vitesse considérablement ralentie pour mesurer une température initiale dudit volume connu de liquide ;

d) l'activation d'un élément chauffant ou réfrigérant (220, 622, 820) en contact thermique avec ledit volume connu de liquide qui est isolé et maintenu immobile ou s'écoule avec une vitesse considérablement ralentie pour ajouter ou retirer une quantité de chaleur connue audit ou dudit volume connu de liquide ;

e) la lecture de la température à partir d'un capteur de température (210, 212, 624, 830, 832) en contact thermique avec ledit volume connu de liquide qui est isolé et maintenu immobile ou s'écoule avec une vitesse considérablement ralentie pour mesurer une température finale dudit liquide après que ladite quantité de chaleur connue a été ajoutée audit ou retirée dudit volume connu de liquide ;

f) la détermination de la valeur d'un coefficient global, qui est égal à la densité multipliée par la capacité calorifique spécifique dudit liquide, à partir dudit volume connu dudit liquide, de ladite quantité de chaleur connue et de la différence entre lesdites températures initiale et finale ;

g) l'activation d'un débitmètre thermique comprenant un élément chauffant ou réfrigérant (220, 622, 820) en contact thermique avec ledit liquide s'écoulant dans ledit conduit (200, 300, 400, 600, 800), ledit élément chauffant ou réfrigérant (220, 622, 820) adapté pour ajouter ou retirer une quantité de chaleur connue audit ou dudit liquide en circulation, et au moins un capteur de température (210, 212, 624, 830, 832) en contact thermique avec ledit liquide s'écoulant dans ledit conduit (200, 300, 400, 600, 800), ledit capteur de température (210, 212, 624, 830, 832) étant adapté pour mesurer la température instantanée dudit fluide en circulation ;

h) la détermination dudit débit volumétrique directement à partir des mesures de la vitesse à laquelle la chaleur est ajoutée audit ou retirée dudit liquide en circulation, la valeur dudit coefficient global et de mesures de la température instantanées, sans l'utilisation de tables de recherche, de coefficients d'un polynôme définissant une courbe de corrélation ou de sources d'informations similaires.

8. Procédé pour mesurer le débit volumétrique d'un liquide ayant des propriétés variables à travers un conduit (200, 300, 400, 600, 800), ledit procédé comprenant les étapes suivantes :

a) la modification d'une section dudit conduit (200, 300, 400, 600, 800) pour former une chambre dans laquelle un volume connu dudit liquide peut être isolé et maintenu immobile ou dans laquelle la vitesse dudit liquide est considérablement réduite ;

b) l'isolement et le maintien immobile ou le ralentissement considérable de la vitesse d'un volume connu de liquide dans ladite chambre ;

c) la lecture de la température à partir d'un capteur de température (210, 212, 624, 830, 832) en contact thermique avec le volume connu de liquide qui est isolé et maintenu immobile ou s'écoule avec une vitesse considérablement ralentie pour mesurer une température initiale dudit volume connu de liquide ;

d) l'activation d'un élément chauffant ou réfrigérant (220, 622, 820) en contact thermique avec ledit volume connu de liquide qui est isolé et maintenu immobile ou s'écoule avec une vitesse considérablement ralentie et la mesure de la quantité de chaleur qui est ajoutée audit ou retirée dudit volume connu de liquide ;

e) la lecture de la température à partir d'un capteur de température (210, 212, 624, 830, 832) en contact thermique avec ledit volume connu de liquide qui est isolé et maintenu immobile ou s'écoule avec une vitesse considérablement ralentie pour déterminer quand une température finale prédéterminée est atteinte, auquel stade ledit élément chauffant ou réfrigérant est désactivé ;

f) la détermination de la valeur d'un coefficient global, qui est égal à la densité multipliée par la capacité calorifique spécifique dudit liquide, à partir dudit volume connu dudit liquide, de ladite quantité de chaleur mesurée et de la différence prédéterminée entre lesdites températures initiale et finale ;

g) l'activation d'un débitmètre thermique comprenant un élément chauffant ou réfrigérant (220, 622, 820) en contact thermique avec ledit liquide s'écoulant dans ledit conduit (200, 300, 400, 600, 800), ledit élément chauffant ou réfrigérant étant adapté pour ajouter ou retirer une quantité de chaleur connue audit ou dudit liquide en circulation, et au moins un capteur de température (210, 212, 624, 830, 832) en contact thermique avec ledit liquide s'écoulant dans ledit conduit (200, 300, 400, 600, 800), ledit capteur de température étant adapté pour mesurer la température instantanée dudit fluide en circulation ;

h) la détermination dudit débit volumétrique directement à partir des mesures de la vitesse à laquelle la chaleur est ajoutée audit ou retirée dudit liquide en circulation, de la valeur dudit coefficient global et de mesures des températures instantanées, sans l'utilisation de tables de recherche, de coefficients d'un polynôme définissant une courbe de corrélation ou de sources d'informations similaires.

9. Procédé selon la revendication 7 ou la revendication 8, dans lequel les étapes de l'étape b à l'étape f sont exécutées simultanément avec l'étape g et l'étape h dans différentes sections du conduit (200, 300, 400, 600, 800).

**10.** Procédé pour mesurer le débit volumétrique d'un liquide ayant des propriétés variables à travers un conduit (200, 300, 400, 600, 800), ledit procédé comprenant les étapes suivantes :

a) la modification d'une section dudit conduit (200, 300, 400, 600, 800) pour former une chambre dans laquelle un volume connu dudit liquide peut être isolé et maintenu immobile ou dans laquelle la vitesse dudit liquide est considérablement réduite ;

b) l'isolement et le maintien immobile ou le ralentissement considérable de la vitesse d'un volume connu de liquide dans ladite chambre ;

c) la lecture de la température à partir d'un capteur de température (210, 212, 624, 830, 832) en contact thermique avec le volume connu de liquide qui est isolé et maintenu immobile ou s'écoule avec une vitesse considérablement ralentie pour mesurer une température initiale dudit volume connu de liquide ;

d) l'activation d'un élément chauffant ou réfrigérant (220, 622, 820) en contact thermique avec ledit volume connu de liquide qui est isolé et maintenu immobile ou s'écoule avec une vitesse considérablement ralentie pour ajouter ou retirer une quantité de chaleur connue audit ou dudit volume connu de liquide ;

e) la lecture de la température à partir d'un capteur de température (210, 212, 624, 830, 832) en contact thermique avec ledit volume connu de liquide qui est isolé et maintenu immobile ou s'écoule avec une vitesse considérablement ralentie pour déterminer quand une température finale prédéterminée est atteinte, auquel stade ledit élément chauffant ou réfrigérant (220, 622, 820) est désactivé ;

f) la reprise de l'écoulement de liquide frais à travers ledit emplacement dans le conduit (200, 300, 400, 600, 800) qui est adapté pour isoler le liquide et pour le maintenir immobile ou pour le forcer à s'écouler avec une vitesse considérablement ralentie et l'activation d'un compteur de temps simultanément à l'instant auquel ledit écoulement de liquide frais est commencé ;

g) la mesure de la période de temps qui s'écoule jusqu'à ce que la température du liquide circulant au niveau dudit emplacement passe de ladite température finale à une valeur de seuil de température ; et

h) la détermination dudit débit volumétrique à partir dudit volume connu dudit liquide et de ladite période de temps mesurée selon l'équation : débit volumétrique égale volume connu divisé par période de temps mesurée.

**11.** Procédé selon une des revendications 7 ou 8, dans lequel ledit procédé est adapté pour mesurer le débit volumétrique d'un liquide à travers un cathéter (910) ou un tube de drainage (918) arrivant d'un patient.

**12.** Procédé selon la revendication 11, dans lequel le liquide est de l'urine.

Fig. 1
Prior Art

Fig. 2

Fig. 3

Fig. 4

Fig. 5A

Fig. 5B

Fig. 5C

Fig. 5D

Fig. 5E

Fig. 6A

Fig. 6B

EP 2 567 199 B1

Fig. 7

Fig. 8

Fig. 9

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6536273 B
- WO 2004100788 A